# EUROPEAN PATENT APPLICATION

(11) **EP 3 940 390 A1**
(43) Date of publication of application: **19.01.2022**
(21) Application number: 20768913.4
(22) Date of filing: 05.03.2020
(51) Int. Cl.: G01N 37/00, G01N 33/543

(54) **METHOD FOR FABRICATING ANALYSIS SUBSTRATE, ANALYSIS SUBSTRATE, AND ANALYSIS UNIT**

(30) Priority: 13.03.2019 JP 2019045916; 13.03.2019 JP 2019045919
(71) Applicant: JVCKENWOOD CORPORATION, Yokohama-shi, Kanagawa 2210022 (JP)
(72) Inventor: TSUJITA, Koji, Yokohama-shi, Kanagawa 221-0022 (JP); ITONAGA, Makoto, Yokohama-shi, Kanagawa 221-0022 (JP); ONO, Masayuki, Yokohama-shi, Kanagawa 221-0022 (JP); SAITO, Atsushi, Yokohama-shi, Kanagawa 221-0022 (JP); IWAMA, Shigehiko, Yokohama-shi, Kanagawa 221-0022 (JP); HORIKOSHI, Katsue, Yokohama-shi, Kanagawa 221-0022 (JP); HASEGAWA, Yuichi, Yokohama-shi, Kanagawa 221-0022 (JP); YAMAMOTO, Masahiro, Yokohama-shi, Kanagawa 221-0022 (JP)
(74) Representative: Wimmer, Hubert
(86) International application number: PCT/JP2020/009410
(87) International publication number: WO 2020/184377

(57) **Abstract**

A method of fabricating a substrate for analysis fixes antibodies (41) that specifically react with specific antigens (52) included in detection target substances (50) to the substrate for analysis (10). The method subjects the substrate for analysis (10) to immersion treatment with a predetermined treatment solution so as to form antibody aggregations (42) in which the antibodies (41) are aggregated at boundary regions between recesses and convex portions on the substrate for analysis (10). The method causes the antigens (52) and the antibodies (41) to react with each other so as to capture the detection target substances (50) on the substrate for analysis (10) by the antibody aggregations (42).

## Description

### [TECHNICAL FIELD]

The present disclosure relates to a method of fabricating a substrate for analysis for analyzing biosamples, a substrate for analysis, and an analysis unit.

### [BACKGROUND ART]

Immunoassays are known that quantitatively analyze disease detection and therapeutic effects by detecting specific antigens or antibodies associated with diseases as biomarkers.

An analysis method disclosed in Patent Literature 1 uses a unit for analysis that includes a plurality of wells, fixes antibodies to a substrate for analysis, causes exosomes as detection target substances to be bound and captured onto the antibodies, and causes microparticles to be bound to the exosomes and quantifies the microparticles, thereby the detection target substances (the exosomes) are indirectly quantified. In the respective steps of fixing the antibodies to the substrate for analysis, capturing the exosomes on the antibodies, and causing the microparticles to be bound to the exosomes, a cleaning solution is injected to the wells through an injection nozzle, and the cleaning solution and other solutions are sucked with a suction nozzle, thereby the wells are cleaned.

Exosomes are membrane vesicles secreted from many kinds of cells and formed of lipid bilayers with a size of about 50 nm to 100 nm, and circulate in body fluids such as blood, saliva, and urine. Exosomes include various kinds of substances on the surfaces or in internal substances thereof, such as proteins, lipids, RNA, mRNA, and micro RNA, which are expected to serve as biomarkers for malignant tumors or many kinds of diseases such as Alzheimer's disease.

The quantitation of the number of exosomes having specific surface antigens derived from a specific disease and the quantitation of the substances included therein from various kinds of exosomes contained in body liquids are presumed to contribute to inspection and early detection of the disease with a higher accuracy.

### [CITATION LIST]

### [PATENT LITERATURE]

Patent Literature 1: Japanese Unexamined Patent Application Publication No. 2014-219384

### [SUMMARY OF THE INVENTION]

To discover a disease earlier, it is important to detect specific exosomes from specimens including a small number of exosomes having specific surface antigens derived from the disease contained in body fluids such as serum at an early stage of the outbreak of the disease. A larger number of exosomes thus needs to be bound to antibodies and captured on the substrate for analysis. It is also important to prevent the exosomes captured on the substrate for analysis from being separated therefrom during cleaning of the wells.

An object of an embodiment is to provide a method of fabricating a substrate for analysis, a substrate for analysis, and an analysis unit capable of reliably capturing detection target substances such as exosomes on the substrate for analysis, and avoiding or reducing separation of the exosomes from the substrate for analysis during cleaning.

A method of fabricating a substrate for analysis according to a first aspect of the embodiment fixes antibodies that react with specific antigens included in detection target substances to the substrate for analysis in which convex portions and recesses alternately arranged, subjects the substrate for analysis to which the antibodies are fixed to immersion treatment with a predetermined treatment solution so as to form antibody aggregations in which the antibodies are aggregated at boundary regions between the recesses and the convex portions, injects a sample solution containing the detection target substances to the substrate for analysis on which the antibody aggregations are formed, and captures the detection target substances on the substrate for analysis.

A substrate for analysis according to a second aspect of the embodiment includes a track region in which convex portions and recesses alternately arranged, and antibody aggregations in which antibodies that react with antigens included in detection target substances are aggregated, the antibody aggregations being formed at boundary regions between the convex portions and the recesses.

A unit for analysis according to a third aspect of the embodiment includes a substrate for analysis having a track region in which convex portions and recesses alternately arranged, and a cartridge provided with a plurality of penetration holes, wherein a plurality of wells are composed of the plural penetration holes and the substrate for analysis serving as a bottom surface of the respective wells, and antibody aggregations in which antibodies that react with antigens included in detection target substances are aggregated are formed at boundary regions between the convex portions and the recesses in the wells.

The method of fabricating the substrate for analysis, the substrate for analysis, and the analysis unit according to the embodiment can reliably capture the detection target substances such as exosomes on the substrate for analysis, and avoid or reduce the separation of the exosomes from the substrate for analysis during cleaning.

### [BRIEF DESCRIPTION OF DRAWINGS]

[Fig. 1] Fig. 1 is a plan view showing an example of a configuration of a unit for analysis.
[Fig. 2] Fig. 2 is a cross-sectional view of the unit for analysis taken along line A-A in Fig. 1.
[Fig. 3] Fig. 3 is a cross-sectional view showing a state in which a cartridge is removed from a substrate for analysis.
[Fig. 4] Fig. 4 is an enlarged perspective view showing a well taken along line B-B in Fig. 1.
[Fig. 5] Fig. 5 is a flowchart showing an example of a method of fabricating the substrate for analysis according to an embodiment.
[Fig. 6] Fig. 6 is a schematic view showing a state in which antibodies are hydrophobically adsorbed to a recess on the substrate for analysis.
[Fig. 7] Fig. 7 is a schematic view showing a state in which antibody aggregations are formed on the recess on the substrate for analysis.
[Fig. 8] Fig. 8 is a scanning electron micrograph (SEM) showing a state in which the antibody aggregations are formed on the recesses on the substrate for analysis.
[Fig. 9] Fig. 9 is a view showing a state in which the antibody aggregations are formed on the substrate for analysis observed with an atomic force microscope.
[Fig. 10] Fig. 10 is a view showing a state in which the antibody aggregations are formed on the substrate for analysis observed with the atomic force microscope.
[Fig. 11] Fig. 11 is a view showing a state in which the antibody aggregations are formed on the substrate for analysis observed with the atomic force microscope.
[Fig. 12] Fig. 12 is a view showing a state in which the antibody aggregations are formed on the substrate for analysis observed with the atomic force microscope.
[Fig. 13] Fig. 13 is a view showing a state in which the antibody aggregations are formed on the substrate for analysis observed with the atomic force microscope.
[Fig. 14] Fig. 14 is a view showing a state in which the antibody aggregations are formed on the substrate for analysis observed with the atomic force microscope.
[Fig. 15] Fig. 15 is a view showing a state in which the antibody aggregations are formed on the substrate for analysis observed with the atomic force microscope.
[Fig. 16] Fig. 16 is a view showing a state in which the antibody aggregations are formed on the substrate for analysis observed with the atomic force microscope.
[Fig. 17] Fig. 17 is a schematic view showing a state in which an exosome is captured on the recess of the substrate for analysis.
[Fig. 18] Fig. 18 is a schematic view showing a state in which an exosome is captured between antibodies and a microparticle on the recess of the substrate for analysis.
[Fig. 19] Fig. 19 is a view showing count values of microparticles indicated as detection sensitivity in Comparative Examples 1 and 2 and Examples 1 and 2.

### [MODES FOR CARRYING OUT THE INVENTION]

### [Unit for Analysis]

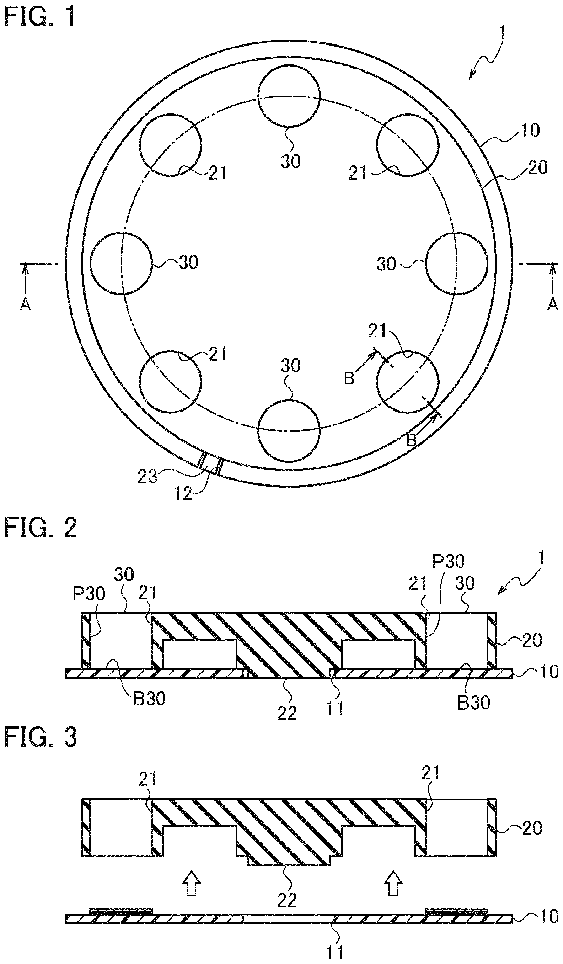

An example of a unit for analysis is described below with reference to Fig. 1 to Fig. 4. As illustrated in Fig. 1, the unit for analysis 1 includes a substrate for analysis 10 and a cartridge 20. Fig. 1 illustrates the unit for analysis 1 as viewed on the cartridge 20 side.

The substrate for analysis 10 has a disc-like shape equivalent to optical discs such as Blu-ray discs (BDs), DVDs, and compact discs (CDs), for example. The substrate for analysis 10 is formed of resin material, such as polycarbonate resin and cycloolefin polymer, used for common optical discs. The substrate for analysis 10 is not limited to the optical disc as described above, and may be any optical disc according to other configurations or conforming to prescribed standards.

Fig. 2 is a cross-sectional view showing the unit for analysis 1 taken along line A-A in Fig. 1. Fig. 3 is a view showing a state in which the cartridge 20 is removed from the substrate for analysis 10. As illustrated in Fig. 1, Fig. 2, or Fig. 3, the substrate for analysis 10 has a central hole 11 and a slit 12. The central hole 11 is provided in the middle of the substrate for analysis 10. The slit 12 is provided at the circumferential part of the substrate for analysis 10. The slit 12 serves as a reference-position defining portion for defining a reference position of the substrate for analysis 10 in a rotating direction.

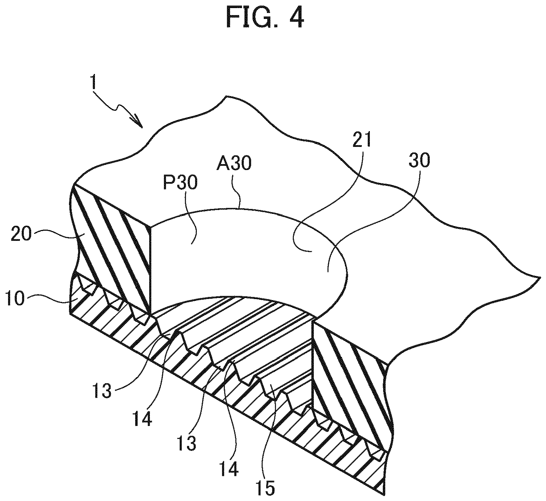

Fig. 4 is a partly enlarged view showing a well 30 taken along line B-B in Fig. 1. The surface of the substrate for analysis 10 includes track regions 15 in which recesses 13 and convex portions 14 alternately arranged in a radial direction. The recesses 13 and the convex portions 14 are formed in a spiral or concentric state from the inner circumference to the outer circumference of the substrate for analysis 10. The recesses 13 correspond to grooves of an optical disc. The convex portions 14 correspond to lands of an optical disc. A track pitch of the substrate for analysis 10 corresponding to a track pitch of an optical disc is 340 nm, for example.

As illustrated in Fig. 1, Fig. 2, or Fig. 3, the cartridge 20 is provided with a plurality of cylindrical penetration holes 21 along the circumferential direction. The penetration holes 21 are arranged at regular intervals such that the respective center points are located on the common circle. The cartridge 20 includes a convex portion 22 provided in the middle, and a convex portion 23 provided at the circumferential part.

The operator, when attaching the cartridge 20 to the substrate for analysis 10, inserts the convex portion 22 to the central hole 11 of the substrate for analysis 10, and inserts the convex portion 23 to the slit 12. This enables the operator to position to align the cartridge 20 and the substrate for analysis 10 with each other.

As illustrated in Fig. 2 or Fig. 4, the unit for analysis 1 includes a plurality of wells 30 formed by the penetration holes 21 of the cartridge 20 and the surface (the respective track regions 15) of the substrate for analysis 10. The wells 30 each have a hollow shape composed of a bottom surface B30, an inner peripheral surface P30, and an open part A30. The surface of the substrate for analysis 10 serves as the bottom surfaces B30 of the respective wells 30. The inner peripheral surface on the inside of the respective penetration holes 21 serves as the inner peripheral surfaces P30 of the respective wells 30.

The open part A30 is located on the opposite side of the bottom surface B30 in the cartridge 20. The wells 30 each serve as a container for storing a solution such as a sample solution, a buffer solution, and a cleaning solution. While Fig. 1 illustrates the eight wells 30, the number of the wells 30 is not limited to eight.

As illustrated in Fig. 3, the cartridge 20 is detachable from the substrate for analysis 10. The detection and the measurement of microparticles for labeling detection target substances are made by use of the substrate for analysis 10 itself separated from the cartridge 20.

### [Method of Fabricating Substrate for Analysis]

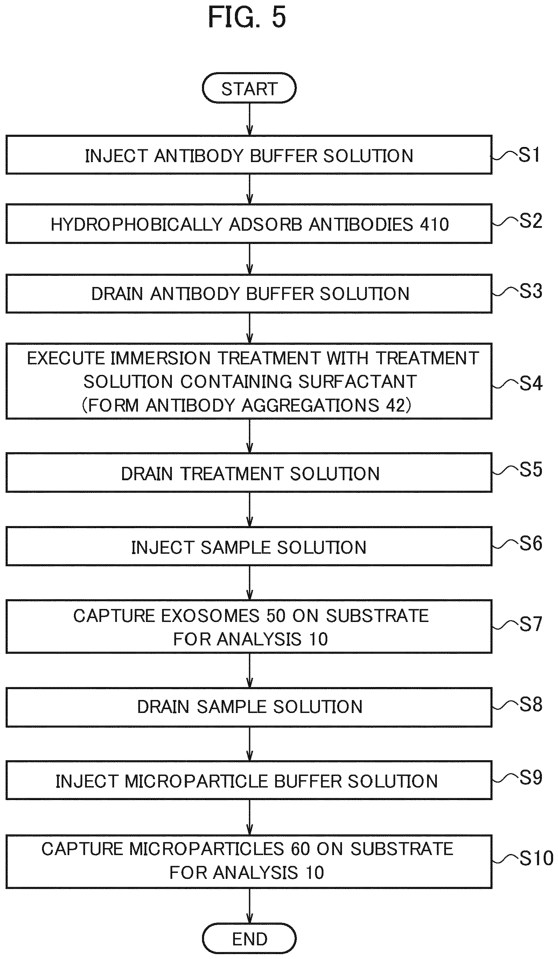

An example of a method of fabricating the substrate for analysis according to an embodiment is described below with reference to the flowchart shown in Fig. 5, and Fig. 6 to Fig. 9. More particularly, a method of fabricating the substrate for analysis 10 to which antibodies are fixed that specifically react with specific antigens included in detection target substances is described below. The embodiment is illustrated below with a case in which the detection target substances are exosomes.

In step S1 in Fig. 5, an injection device injects an antibody buffer solution to the wells 30. The antibody buffer solution to be used may be a carbonate-bicarbonate buffer solution including antibodies 41 (first antibodies) that specifically react with specific antigens in exosomes. In step S2, a reaction device incubates the unit for analysis 1 in which the antibody buffer solution is injected to the wells 30. The incubation causes part of the antibodies 41 that has hydrophobicity to be adsorbed to part of the substrate for analysis 10 that has hydrophobicity.

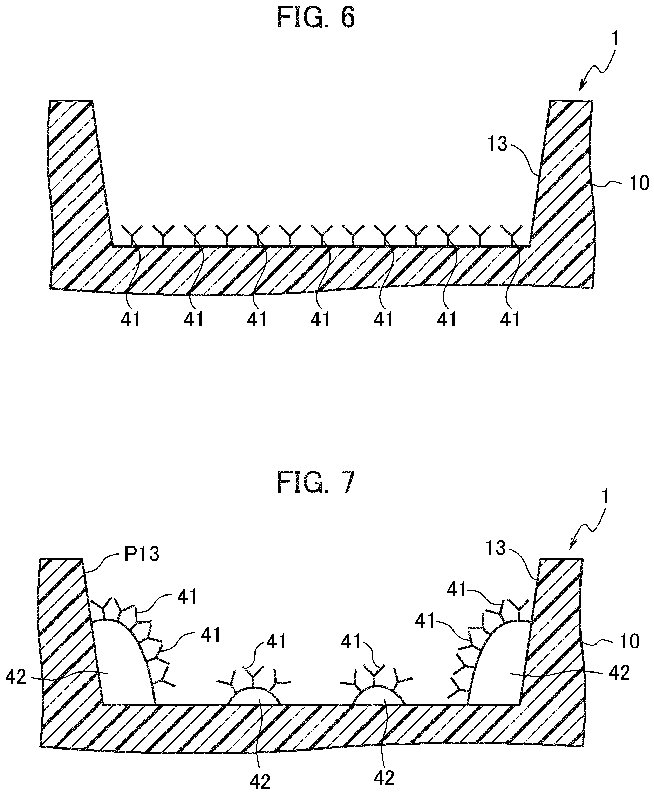

As illustrated in Fig. 6, the antibodies 41 are hydrophobically adsorbed to a region corresponding to the respective bottom surfaces B30 of the wells 30 on the substrate for analysis 10. The antibodies 41 are fixed to the substrate for analysis 10. Fig. 6 is an enlarged cross-sectional view of the recess 13 on the track region 15 of the substrate for analysis 10, and schematically illustrates a state in which the antibodies 41 are hydrophobically adsorbed to the recess 13.

In step S3, a cleaning device drains the antibody buffer solution from the wells 30. In step S4, the injection device injects a treatment solution containing a surfactant to the wells 30, and executes immersion treatment at a set temperature for a set period. The injection device may execute the immersion treatment while keeping the unit for analysis 1 stopped, or may execute the immersion treatment while keeping the unit for analysis 1 shaken.

The treatment solution to be used can be a buffer solution for bioassay. A preferred example of the buffer solution for bioassay is phosphate-buffer saline (PBS). A nonionic surfactant can be used as the surfactant contained in the treatment solution. A preferred example of the nonionic surfactant is polyoxyethylene sorbitan monolaurate (Tween 20) or octylphenol ethoxylate (Triton X-100).

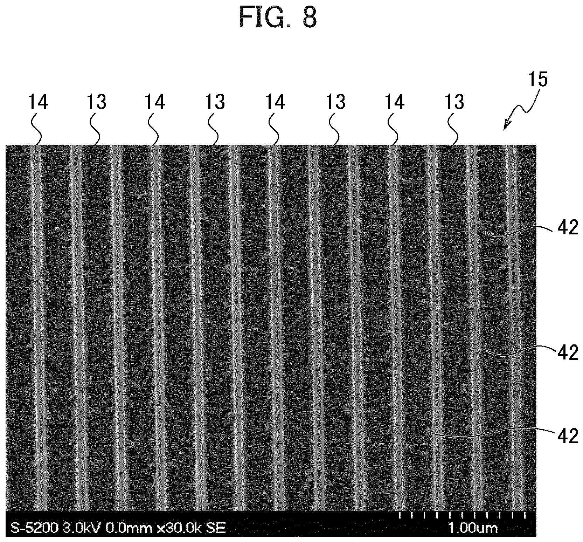

As illustrated in Fig. 7 or Fig. 8, the immersion treatment executed in step S4 leads the antibodies 41 hydrophobically adsorbed to the substrate for analysis 10 to be aggregated so as to form antibody aggregations 42. That is, each antibody aggregation 42 is composed of the plural antibodies 41. Fig. 7 corresponding to Fig. 6 schematically illustrates the antibody aggregations 42 in which the antibodies 41 are aggregated. Fig. 8 is an image of the track region 15 observed by a scanning electron microscope (SEM) corresponding to the bottom surface B30 of the well 30 on the substrate for analysis 10. The antibody aggregations 42 are mainly formed on the recesses 13 of the substrate for analysis 10. In particular, the antibody aggregations 42 in which the antibodies 41 are aggregated are mainly formed from the vicinity of the boundaries between the recesses 13 and the convex portions 14 to inner side surfaces P13 of the recesses 13. Namely, the antibody aggregations 42 are formed at the boundary regions between the convex portions 14 and the recesses 13, and are also formed in contact with the respective inner side surfaces P13 of the recesses 13.

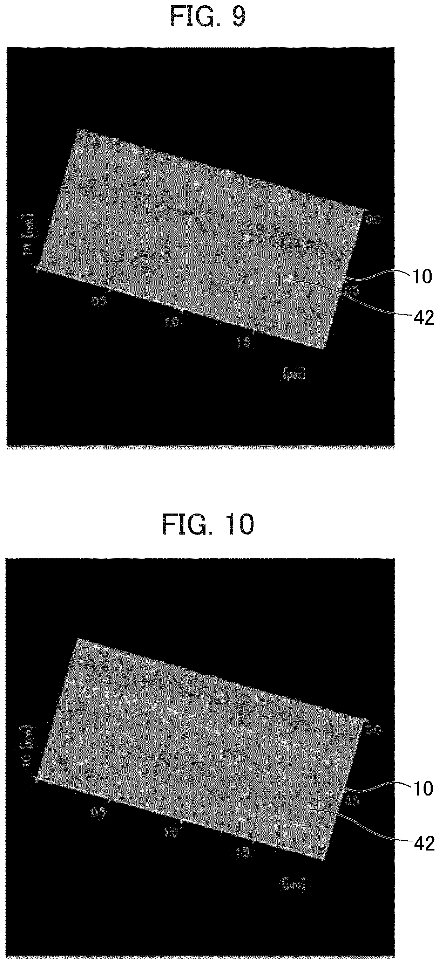

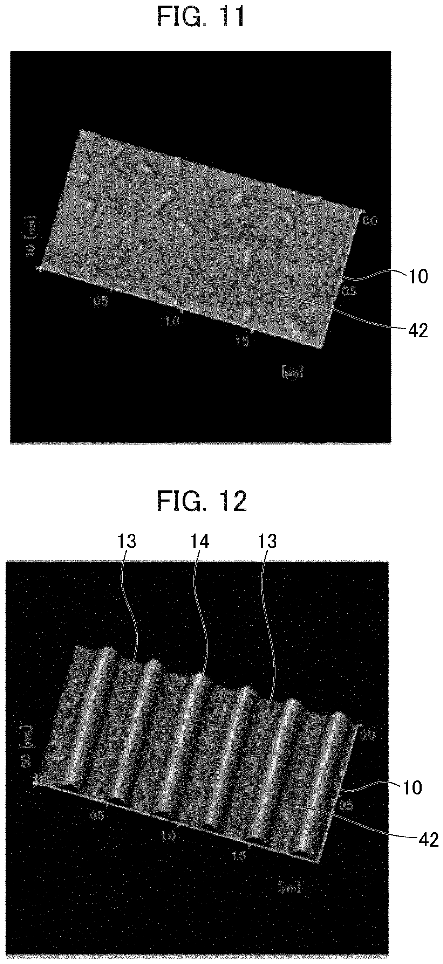

A relationship between a concentration of the surfactant contained in the treatment solution and the antibody aggregations 42 formed on the substrate for analysis 10 is described below with reference to Fig. 9 to Fig. 14. Fig. 9 to Fig. 11 show the substrate for analysis 10 of comparative examples not provided with the track regions 15, and Fig. 12 to Fig. 14 show the substrate for analysis 10 of the present examples provided with the track regions 15.

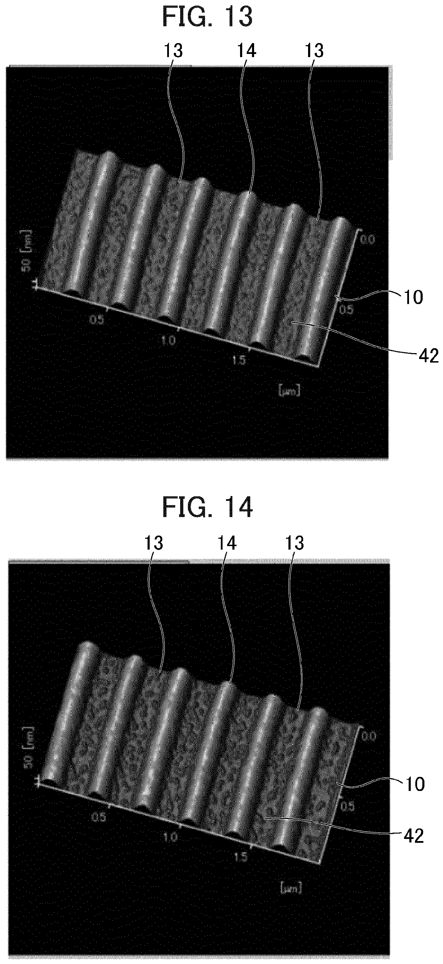

Fig. 9 and Fig. 12 are micrographs showing a case in which the antibody aggregations 42 are formed on the substrate for analysis 10 through the immersion treatment with the treatment solution containing the surfactant having a first concentration. Fig. 10 and Fig. 13 are micrographs showing a case in which the antibody aggregations 42 are formed on the substrate for analysis 10 through the immersion treatment with the treatment solution containing the surfactant having a second concentration higher than the first concentration. Fig. 11 and Fig. 14 are micrographs showing a case in which the antibody aggregations 42 are formed on the substrate for analysis 10 through the immersion treatment with the treatment solution containing the surfactant having a third concentration higher than the second concentration.

As shown in Fig. 9 to 11, the distribution and the size of the antibody aggregations 42 tend to be influenced by the concentration of the surfactant contained in the treatment solution when the track regions 15 are not provided on the substrate for analysis 10, and are thus difficult to adjust with a high accuracy. As shown in Fig. 12 to Fig. 14, the distribution and the size of the antibody aggregations 42 are not easily influenced by the concentration of the surfactant contained in the treatment solution when the track regions 15 are provided on the substrate for analysis 10, and thus can be adjusted accurately.

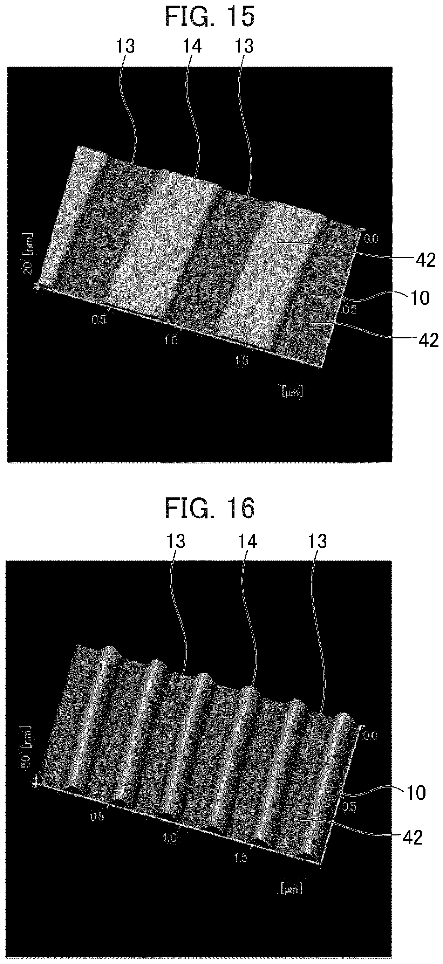

A relationship between a width of the respective recesses 13 and the antibody aggregations 42 formed on the substrate for analysis 10 is described below with reference to Fig. 15 and Fig. 16. Fig. 15 is a micrograph showing a case in which the pitch between the recesses 13 and the convex portions 14 in the radial direction of the substrate for analysis 10 is 740 nm, and the width of the respective recesses 13 is 370 nm. Fig. 16 is a micrograph showing a case in which the pitch between the recesses 13 and the convex portions 14 in the radial direction of the substrate for analysis 10 is 340 nm, and the width of the respective recesses 13 is 220 nm.

Increasing the width of the recesses 13 more than the width of the convex portions 14 increases the density of the antibodies 41 adjacent to the recesses 13 more than the density of the antibodies 41 adjacent to the convex portions 14, thereby the antibody aggregations 42 can be selectively formed in the recesses 13. The width of the convex portions 14 is thus smaller than the width of the recesses 13.

When the pitch between the recesses 13 and the convex portions 14 in the radial direction of the substrate for analysis 10 is 340 nm, the width of the recesses 13 in the radial direction of the substrate for analysis 10 is preferably set in a range of 150 nm to 250 nm, and the depth of the recesses 13 (the height of the convex portions 14) is preferably set in a range of 20 nm to 100 nm, and more preferably in a range of 65 nm to 70 nm in order to form the antibody aggregations 42 mainly in the recesses 13.

The antibody aggregations 42 preferably have a size in a range of 5 nm to 40 nm, and more preferably in a range of 10 nm to 20 nm. The size of the antibody aggregations 42 can be adjusted depending on the concentration of the surfactant contained in the treatment solution and the immersion time. For example, executing the immersion treatment for a period in a range of one to two hours while using Tween 20 as the surfactant and setting the concentration of the surfactant contained in the treatment solution to 0.05 wt% can adjust the size of the antibody aggregations 42 to about 10 nm to 20 nm.

In step S5, the cleaning device drains the treatment solution from the wells 30. The cleaning device cleans the inside of the wells 30 with a cleaning solution after the removal of the treatment solution. The cleaning solution may be either pure water or a solution containing a surfactant. When the cleaning solution contains a surfactant, the immersion time in step S4 is longer than the cleaning time in step S5, and the concentration of the surfactant contained in the treatment solution is higher than or equal to the concentration of the surfactant contained in the cleaning solution.

In step S6, the injection device injects, to the wells 30, a sample solution that contains or has a probability of containing exosomes as a target to be detected. The following example is illustrated with a case in which the sample solution contains exosomes as a target to be detected for brevity.

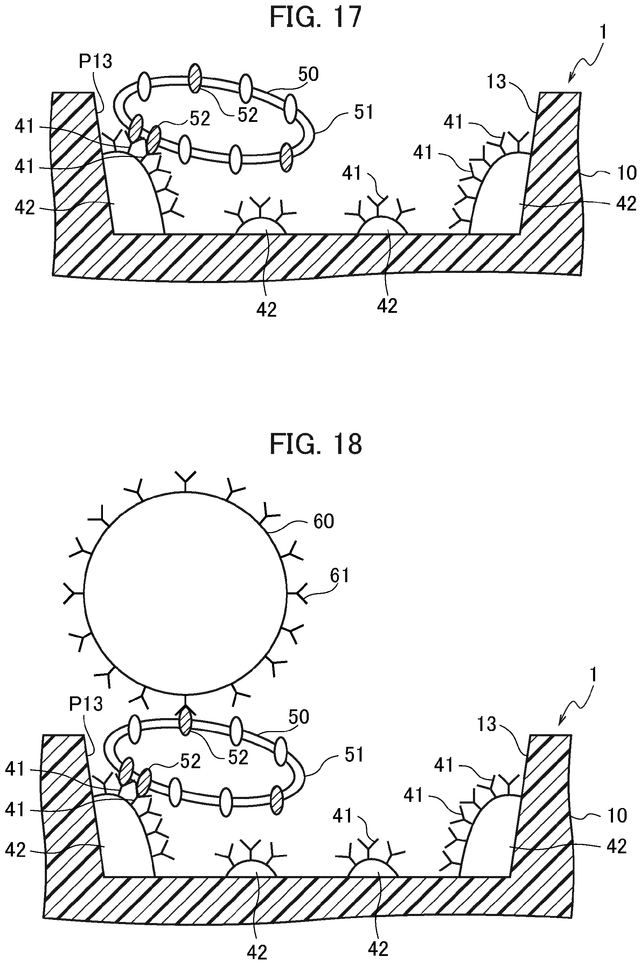

As illustrated in Fig. 17, an exosome 50 is covered with a lipid bilayer membrane 51. Fig. 17 corresponds to Fig. 7. The lipid bilayer membrane 51 contains a plurality of transmembrane proteins. The number of the transmembrane proteins and the position in the lipid bilayer membrane 51 vary depending on the type of the exosomes. Fig. 17 schematically illustrates an example of the exosome 50 in which the lipid bilayer membrane 51 contains nine transmembrane proteins, and four of the membrane proteins are antigens 52 as a target to be detected.

In step S7, the reaction device incubates the unit for analysis 1 at a set temperature for a set time. The antigens 52 in the exosomes 50 contained in the sample solution specifically react with the antibodies 41 included in the antibody aggregations 42. The incubation leads the exosomes 50 to be captured on the substrate for analysis 10.

Since the antibody aggregations 42 include the plural antibodies 41, the antibody aggregations 42 can efficiently capture the exosomes 50 contained in the sample solution. As illustrated in Fig. 17, the plural antigens 52 in the exosome 50 and the plural antibodies 41 in the antibody aggregations 42 sometimes specifically react with each other. The antibody aggregations 42 are mainly formed in contact with the inner side surfaces P13 of the recesses 13, thereby the exosomes 50 can be strongly captured in the vicinity of the inner side surfaces P13 of the recesses 13 as compared with a case without the antibody aggregations 42 formed.

In step S8, the cleaning device drains the sample solution from the wells 30. The cleaning device cleans the inside of the wells 30 with a cleaning solution after the removal of the sample solution. The exosomes 50 are mainly captured adjacent to the inner side surfaces P13 of the recesses 13, and are not thus easily influenced by the flow of the cleaning solution. Since the exosomes 50 are not easily removed from the substrate for analysis 10, the separation of the exosomes 50 captured on the substrate for analysis 10 thus can be avoided.

In step S9, the injection device injects a buffer solution containing microparticles (referred to below as a "microparticle buffer solution") to the wells 30. As illustrated in Fig. 18, the surface of the microparticles 60 is provided with a plurality of antibodies 61 (second antibodies) that specifically react with the antigens 52 in the exosome 50.

In step S10, the reaction device incubates the unit for analysis 1 at a set temperature for a set time. The antibodies 61 of the microparticles 60 contained in the microparticle buffer solution specifically react with the antigens 52 in the exosomes 50. The incubation leads the microparticles 61 to be captured on the substrate for analysis 10, and leads the exosomes 50 to be captured on the substrate for analysis 10 in a sandwiching way between the antibodies 41 and the microparticles 60. The exosomes 50 as detection target substances are thus arranged between the antibody aggregations 42 and the microparticles 60.

The exosomes 50 are labeled by the microparticles 60. The analysis device quantifies (counts) the number of the microparticles 60 captured on the substrate for analysis 10, thereby the number of the exosomes 50 can be quantified (counted) indirectly. The operator may execute at least any of the steps S1 to S 10, or may execute part of the treatment in the steps. The antibodies 61 formed on the surfaces of the microparticles 60 may be either the antibodies that specifically react with the antigens 52 in the exosomes 50, or antibodies or proteins that specifically react with other antigens or proteins expressed on the surfaces of the exosomes 50.

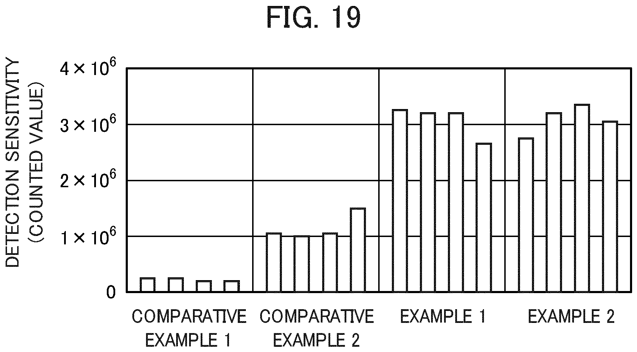

The functional effects of the method of fabricating the substrate for analysis according to the present embodiment are described below with reference to Fig. 19. The vertical axis in Fig. 19 shows the counted values of the microparticles 60 in the wells 30 indicated as detection sensitivity of the exosomes 50.

Comparative Examples 1 and 2 shown in Fig. 19 each indicate the detection sensitivity in a case of not executing the immersion treatment with the treatment solution containing the surfactant in step S4 according to the present embodiment. In particular, Comparative Example 1 indicates the detection sensitivity in the case in which the wells 30 are cleaned with the cleaning solution not containing the surfactant after step S3 according to the present embodiment. Comparative Example 2 indicates the detection sensitivity in the case in which the wells 30 are cleaned with the cleaning solution containing the surfactant after step S3 according to the present embodiment. The results revealed that the cleaning of the inside of the wells 30 with the cleaning solution containing the surfactant after step S3 improves the detection sensitivity to some extent, as compared with the case of cleaning the inside of the wells 30 with the cleaning solution not containing the surfactant, due to effect of the surfactant contained in the cleaning solution.

Examples 1 and 2 shown in Fig. 19 each indicate the detection sensitivity in a case of executing the immersion treatment with the treatment solution containing the surfactant in step S4 according to the present embodiment. In particular, Example 1 corresponds to Comparative Example 1, and indicates the detection sensitivity in the case of cleaning the wells 30 with the cleaning solution not containing the surfactant after executing the immersion treatment with the treatment solution containing the surfactant in step S4 according to the present embodiment. Example 2 corresponds to Comparative Example 2, and indicates the detection sensitivity in the case of cleaning the wells 30 with the cleaning solution containing the surfactant after executing the immersion treatment with the treatment solution containing the surfactant in step S4 according to the present embodiment.

The detection sensitivity in Example 1 is about eight times as high as that in Comparative Example 1, and the detection sensitivity in Example 2 is about three times as high as that in Comparative Example 2. The results revealed that Examples 1 and 2 both exhibit the detection sensitivity that is greatly improved as compared with Comparative Examples 1 and 2.

The method of fabricating the substrate for analysis, the substrate for analysis, and the analysis unit according to the present embodiment cause the antibodies 41 that specifically react with the specific antigens 52 in the exosomes 50 as the detection target substances to be hydrophobically adsorbed to the recesses 13 provided on the track regions 15 on the substrate for analysis 10. The substrate for analysis 10 is subjected to the immersion treatment with the treatment solution containing the surfactant so as to form the antibody aggregations 42 at the boundary regions between the recesses 13 and the convex portions 14. The treatment solution used in the immersion treatment may be any treatment solution containing any other substance other than the surfactant that has the effect of leading the antibodies 41 to be aggregated.

The method of fabricating the substrate for analysis, the substrate for analysis, and the analysis unit according to the present embodiment lead the exosomes 50 in the sample solution to be captured on the antibody aggregations 42 formed at the boundaries between the recesses 13 and the convex portions 14 provided on the track regions 15 on the substrate for analysis 10, thereby the separation of the exosomes 50 from the substrate for analysis 10 in the cleaning step can be avoided or reduced.

It should be understood that the present invention is not intended to be limited to the respective embodiments described above, and various modifications will be apparent to those skilled in the art without departing from the scope of the present invention.

The present disclosure relates to the subject matter of Japanese Patent Application No. P2019-045916 and Japanese Patent Application No. P2019-045919 filed on March 13, 2019, the entire contents of which are incorporated herein by reference.

## Claims

1. A method of fabricating a substrate for analysis, the method comprising:
fixing antibodies that react with specific antigens included in detection target substances to the substrate for analysis in which convex portions and recesses alternately arranged;
subjecting the substrate for analysis to which the antibodies are fixed to immersion treatment with a predetermined treatment solution so as to form antibody aggregations in which the antibodies are aggregated at boundary regions between the recesses and the convex portions;
injecting a sample solution containing the detection target substances to the substrate for analysis on which the antibody aggregations are formed; and
capturing the detection target substances on the substrate for analysis.

2. The method of fabricating the substrate for analysis according to claim 1, wherein:
the antibodies are fixed to the substrate for analysis by hydrophobic adsorption; and
the antibody aggregations are formed by use of a surfactant as the predetermined treatment solution.

3. The method of fabricating the substrate for analysis according to claim 1 or 2, further comprising:
fixing, to the detection target substances, microparticles provided with the antibodies or other antibodies different from the antibodies on surfaces of the microparticles; and
arranging the detection target substances between the antibody aggregations and the microparticles.

4. A substrate for analysis comprising:
a track region in which convex portions and recesses alternately arranged; and
antibody aggregations in which antibodies that react with antigens included in detection target substances are aggregated, the antibody aggregations being formed at boundary regions between the convex portions and the recesses.

5. The substrate for analysis according to claim 4, wherein the recesses have a wider width than the convex portions.

6. A unit for analysis comprising:
a substrate for analysis having a track region in which convex portions and recesses alternately arranged; and
a cartridge provided with a plurality of penetration holes,
wherein a plurality of wells are composed of the plural penetration holes and the substrate for analysis serving as a bottom surface of the respective wells, and
antibody aggregations in which antibodies that react with antigens included in detection target substances are aggregated are formed at boundary regions between the convex portions and the recesses in the wells.
